# EUROPEAN PATENT APPLICATION

(11) **EP 4 772 605 A1**
(43) Date of publication of application: **08.07.2026**
(21) Application number: 24921236.6
(22) Date of filing: 27.05.2024
(51) Int. Cl.: C12M 1/34, C12M 1/00, C12Q 1/6837, C12Q 1/686, C12Q 1/6844, C12N 15/10, B01L 3/00

(54) **FULLY INTEGRATED NUCLEIC ACID TESTING CHIP BASED ON SILICON FILM METHOD**

(30) Priority: 02.02.2024 CN 202410150037
(71) Applicant: ingeDx Technologies Co., Ltd., Suzhou, Jiangsu 215000 (CN)
(72) Inventor: LI, Xiaoning, Suzhou, Jiangsu 215000 (CN); ZHOU, Peng, Suzhou, Jiangsu 215000 (CN); MA, Junjie, Suzhou, Jiangsu 215000 (CN)
(74) Representative: RCF - Protecting Innovation, S.A.
(86) International application number: PCT/CN2024/095395
(87) International publication number: WO 2025/161194

(57) **Abstract**

Provided is a flexible thin-film microfluidic chip, comprising a laminated film (1000), at least one fixed bonding region, a plurality of functional chambers, fluid channels (1110), at least one valve region and at least one fluid inlet. The fixed bonding region, functional chambers, fluid channels (1110) and valve region are all distributed within the laminated film (1000). The sizes and shapes of the functional chambers and fluid channels (1110) are defined by the boundary of the fixed bonding region. The fluid inlet is connected to at least one functional chamber, and is sealed after fluid introduction. The flexible thin-film microfluidic chip is partitioned into at least three functional regions: a nucleic acid extraction region, a nucleic acid quantification region, and a nucleic acid detection region. Each functional region comprises at least one functional chamber. The functional chamber(s) within the nucleic acid quantification region comprises at least one volumetric chamber (1313).

## Description

### Technical Field

The present invention relates to an integrated nucleic acid detection chip based on silica membrane method and belongs to the technical field of microfluidic chips.

### Background Art

Since the advent of polymerase chain reaction (PCR) technology, DNA amplification for genetic analysis has become a widely used technique. However, conventional PCR tube-based amplification devices suffer from inherent structural limitations, including high energy consumption, long detection time, cross-contamination, and the inability to perform real-time on-site testing. As a result, traditional PCR devices are increasingly unable to meet application requirements across diverse scenarios.

With the emergence of microfluidics technology, its advantages-including high integration, miniaturization, fully enclosed processing, and suitability for a wide range of application scenarios-have attracted substantial attention. Over the past decade, a large number of microfluidic chips featuring different structures and thermal-cycling mechanisms have been developed and widely applied to pathogen detection. The advantages of microfluidic detection products in point-of-care testing (POCT) settings have been fully demonstrated worldwide during the COVID-19 pandemic.

Traditional microfluidic chips are primarily fabricated using cured PDMS (polydimethylsiloxane). Due to the soft lithography-based fabrication process, PDMS chips are highly suitable for proof-of-concept research and small-batch prototyping in laboratories. However, this manufacturing method is not suitable for large-scale production, especially automated mass production. Moreover, as a rubber-based material, PDMS is not widely accepted in the industry for manufacturing in-vitro diagnostic (IVD) products.

Patent CN2022109249601 discloses a microfluidic chip fabricated from flexible films, which can be used for cell lysis and nucleic acid extraction. The chip is formed by stacking a first flexible film and a second flexible film, with multiple interconnected functional chambers arranged between the films. Fluid channels may be provided between the chambers. Rapid fluid flow is achieved through short microchannels to enhance lysis efficiency, and magnetic beads are used to perform nucleic acid extraction.

Patent CN2008801038390 discloses a flexible-sheet-based multi-chamber container for nucleic acid amplification. It employs magnetic-bead-based nucleic acid extraction and elution. To facilitate efficient movement of magnetic beads or fluids between chambers, the chambers are directly connected over the shortest possible distance or connected via valve structures.

Patent CN2022110178734 discloses a flexible thin-film-based microfluidic chip for nucleic acid amplification. The chip comprises a composite film, fixed bonding areas, multiple functional chambers, single-use valves, and liquid- and gas-blocking fluid channels. Within the functional chambers and the liquid- and gas-blocking fluid channels, the first flexible film and the second flexible film can switch between separated and adhered states under applied pressure. When the films separate inside the liquid- and gas-blocking fluid channels to form a fluid pathway, the pathway allows liquid to pass through while preventing isolated gas bubbles from being carried forward.

Magnetic-bead-based nucleic acid extraction is currently the most widely used method. The magnetic beads typically have a core composed of minute (20-30 nm) magnetic iron oxides such as magnetite (Fe₃0₄). Unlike conventional magnetic materials, the extremely small size of these particles confers superparamagnetic properties. They become magnetized only under the influence of a strong magnetic field, and do not attract each other when the magnetic field is removed, thereby effectively preventing aggregation caused by bead clustering. This characteristic makes magnetic beads an ideal material for macromolecule separation or surface-specific adsorption via surface chemistry in complex biological samples. Different surface modifications result in distinct chemical properties, enabling magnetic-bead-based extraction to be widely used in the separation and purification of nucleic acids, proteins, and other biomolecules in fields such as in-vitro diagnostics (IVD), gene sequencing, environmental protection, and food safety. When used for nucleic acid adsorption, the bead surface is typically coated with silicon dioxide (SiO₂). Under suitable salt concentration, pH, and nucleic acid binding buffer conditions, magnetic beads can efficiently adsorb nucleic acids in the reaction mixture and, when exposed to a strong magnetic field, can be collected at a specific location in the reaction vessel. This allows the waste liquid to be removed while the beads remain immobilized. Consequently, during nucleic acid extraction, magnetic beads can undergo repeated cycles of enrichment and release, enabling removal of impurities adsorbed nonspecifically on the bead surface and ultimately yielding purified nucleic acid templates upon elution.

When the magnetic-bead extraction workflow is adapted for use in POCT microfluidic devices, the high degree of integration of such devices, the micro-scale dimensions of the fluidic structures, and stringent cost-control requirements pose significant challenges for designing consumable extraction structures, manufacturing processes, and coordination with the instrument's control modules.

Another commonly used nucleic acid extraction method-though with more limited universality-is the silica membrane spin-column method. Like the magnetic-bead method, the material responsible for adsorbing nucleic acids in the spin-column method is also SiO₂. The extraction process relies on nucleic acid binding and release under different salt concentrations and pH conditions to achieve nucleic acid purification. A fundamental requirement of this method is that, under centrifugal force, the reaction mixture must pass through the silica membrane and exit into a waste liquid container, ensuring thorough contact between the sample nucleic acids and the SiO₂ matrix to complete the adsorption process. Clearly, during this method, the lysate, wash buffer, and elution buffer all pass uniformly through the membrane in a direction perpendicular to the membrane plane under centrifugal force, enabling efficient and rapid nucleic acid extraction.

Another implementation of the silica membrane method replaces centrifugal force with vacuum suction. In this configuration, multiple spin columns are sealed onto a vacuum chamber with multiple outlets. After sample or buffer loading, a negative pressure is applied to the vacuum chamber. Under the vacuum force, the liquid inside each spin column is drawn through the membrane into the vacuum chamber, thereby achieving nucleic acid adsorption and washing.

In this passage mode, the adsorption gradient of the silica membrane is inversely proportional to its thickness. Since the membrane can typically be made very thin, it provides a large working surface area with only a slight decrease in gradient, resulting in high adsorption efficiency. However, achieving nucleic acid adsorption, washing, elution, and subsequent nucleic acid detection within a closed, two-dimensional flexible thin-film microfluidic chip using a silica membrane-based method presents challenges. If the silica membrane is fixed inside the microfluidic channels of a flexible thin-film chip, and the sample solution, wash buffer, and elution buffer with appropriate salt concentrations are sequentially passed through, then in principle, nucleic acid extraction and purification from complex samples can be achieved. In practice, however, difficulties arise. On one hand, because the flexible thin-film microfluidic chip has a two-dimensional planar structure, the microfluidic channel embedding the silica membrane does have some thickness, but its cross-sectional dimensions are far smaller than the planar cross-section typically used in conventional spin-column silica membrane workflows. Under these conditions, in order to ensure sufficient adsorption of nucleic acids onto the silica membrane, the length of the flow path containing the membrane must be significantly greater than the membrane thickness that the liquid travels through in a spin column. As a result, the fluidic resistance generated in the process increases substantially as more liquid passes through the membrane. On the other hand, both the materials used and the fabrication methods of flexible thin-film microfluidic chips impose limits on the device's mechanical flexibility. Under continuous or increasing pressure, the microchannels may undergo destructive deformation, leading to experimental failure. Therefore, the conventional spin-column silica membrane method cannot be directly applied to flexible thin-film microfluidic chips.

On the other hand, during nucleic acid extraction, the saliva sample undergoes several steps, including cell lysis and binding, nucleic acid adsorption and purification, and nucleic acid elution and collection. During nucleic acid detection, the extracted nucleic acid sample needs to undergo steps such as mixing with the PCR reaction solution and qPCR amplification.

For example, a patent document in the prior art, CN202211170669.6, discloses an integrated nucleic acid extraction microfluidic chip cartridge and a nucleic acid extraction and detection method. The integrated nucleic acid extraction microfluidic chip cartridge comprises a microfluidic chip body, a driving assembly, and a reaction assembly. The microfluidic chip body comprises a plurality of chambers that can be in communication with one another, valve bodies for controlling communication or blocking between the chambers, and a driving assembly for driving the flow of liquid between the respective chambers. The method for nucleic acid extraction and detection using the integrated nucleic acid extraction microfluidic chip cartridge comprises the following steps: sample loading, lysis, nucleic acid capture, washing, elution, PCR mixture preparation, PCR amplification, and the like.

However, in this existing technology, during the elution, PCR mixing, and PCR amplification steps, the nucleic acids are first eluted and mixed with the elution buffer. The eluted nucleic acid solution is then mixed with PCR reagents, and the PCR reaction chamber is heated and optically monitored to complete nucleic acid detection. During the mixing of the eluted nucleic acid solution with the PCR reagents, the volume of the nucleic acid-containing elution buffer cannot be accurately quantified-or cannot be quantified at all-which easily leads to detection failure or inaccurate results. For example, the volume of elution buffer containing nucleic acids that corresponds to the intended stoichiometry for reaction with the PCR reagents is defined as the predetermined volume. If the volume of the elution buffer that reacts with the PCR reagent is greater than a predetermined volume, the nucleic acid quantification result is likely to be higher than the actual result. Conversely, if the volume of the elution buffer that reacts with the PCR reagent is less than the predetermined volume, the nucleic acid quantification result is likely to be lower than the actual result.

### Summary of the Invention

To address the above issues in existing technologies, the main objective of the invention is to provide a flexible thin-film microfluidic chip and a fully integrated method for quantitative nucleic acid detection. This method enables nucleic acid extraction using a silica membrane-based approach, allows simple and efficient quantification of the nucleic acid elution volume, and achieves on-chip real-time PCR amplification and detection. The system integrates nucleic acid extraction, nucleic acid quantification, PCR amplification, and fluorescence detection into a single workflow, thereby expanding the application scope of current microfluidic technologies.

The present invention is implemented through the following technical solution:
A flexible thin-film microfluidic chip, comprising a laminated film, at least one fixed bonding region, multiple functional chambers, fluid channels, at least one valve region, and at least one fluid inlet. The fixed bonding region, functional chambers, fluid channels, and valve region are all distributed within the laminated film. The size and shape of the functional chambers and fluid channels are defined by the boundaries of the fixed bonding region. The fluid inlet is connected to at least one functional chamber and is sealed after fluid introduction; The valve region may optionally be arranged between the functional chambers and the fluid channels, and an irreversible, single-use valve structure is distributed within the valve region.

The laminated film comprises a first film and a second film laminated together, wherein at least one of the first and second films is a flexible film. Within the fixed bonding region, the first film and the second film are irreversibly bonded.

The first and second films corresponding to the functional chambers can switch between two states-separated and attached-under applied pressure. When the first and second films within a functional chamber are separated, the chamber can accommodate fluid.

The first and second films corresponding to the fluid channels can also switch between separated and attached states under applied pressure. When fluid flows out of a functional chamber under pressure, the first and second films of the fluid channel separate under the fluid pressure, allowing the fluid to pass through the fluid channel and move from one functional chamber to another.

The flexible thin-film microfluidic chip comprises at least three sequentially connected functional regions: a nucleic acid extraction region, a nucleic acid quantification region, and a nucleic acid detection region, wherein the nucleic acid quantification region is located between the nucleic acid extraction region and the nucleic acid detection region. Each functional region comprises at least one functional chamber.

Furthermore, the functional chambers in the nucleic acid quantification zone comprise at least a volumetric chamber and an auxiliary volumetric chamber. The volumetric chamber is respectively connected, at its two ends, to the functional chambers of the nucleic acid extraction zone and the nucleic acid detection zone. During the flow of the nucleic acid extract from the nucleic acid extraction zone toward the nucleic acid detection zone, the extract successively flows into the volumetric chamber and the auxiliary volumetric chamber. After the volumetric chamber is fully filled, any extract exceeding its capacity flows into the auxiliary volumetric chamber.

The principle of nucleic acid quantification in this invention is as follows: the pressure difference between the inlet and outlet of the volumetric chamber is defined as △P, which determines the volume at which the first and second films separate inside the quantification chamber. When △P is a fixed value and greater than the longitudinal elastic deformation force of the flexible films, the two films separate, and the resulting separation volume becomes a fixed volume. This fixed volume represents the quantification volume under those conditions.

The value of △P is determined by the pressure difference between the inlet and outlet. The outlet pressure is fixed, determined by the geometry of the outlet channel. The inlet pressure can be adjusted by changing the force applied to compress the second elution chamber. When greater force is applied, the inlet pressure increases, resulting in a larger △P and thus a larger quantification volume. Conversely, applying less force reduces the inlet pressure, resulting in a smaller △P and a smaller quantification volume.

In this way, different quantification volumes can be achieved without altering the structural design of the quantification chamber.

In other words, the capacity of the volumetric chamber can be adjusted through controlling the inlet-outlet pressure difference, without modifying the physical structure of the quantification chamber.

The nucleic acid extraction region includes at least one silica membrane chamber, which is used to capture nucleic acids in the liquid.

The maximum width of the silica membrane chamber is greater than the width of the fluid channel, and a silica membrane matching its shape is placed flat within the chamber.

The functional chambers of the nucleic acid extraction zone are connected to the silica membrane chamber through fluid channels, which allow controlled, low-speed flow of fluid therethrough.

Furthermore, the functional chambers in the nucleic acid extraction zone comprise at least one sample chamber, at least one wash buffer chamber, at least one waste liquid chamber, and at least two elution chambers. The two elution chambers are a first elution chamber and a second elution chamber. The volumetric chamber is connected to the second elution chamber either directly or via a single-use valve.

Furthermore, the functional chambers in the nucleic acid extraction zone comprise one sample chamber, two wash buffer chambers, one waste liquid chamber, and two elution chambers. The two wash buffer chambers are a first wash buffer chamber and a second wash buffer chamber.

Furthermore, that the fluid channels in the nucleic acid extraction zone are divided into sections by the silica membrane chamber, and the sections are classified according to the functional chambers to which they are connected. The sections include at least a first fluid channel and a second fluid channel, wherein the first fluid channel is connected to the sample chamber and the second fluid channel is connected to the waste liquid chamber.

Furthermore, the functional chambers in the nucleic acid extraction zone further comprise at least one lysis buffer chamber and at least one binding buffer chamber. The lysis buffer chamber is located between the sample chamber and the binding buffer chamber and is connected to them respectively via single-use valves. The liquid inlet interface is connected to the sample chamber.

The manner in which the functional chambers are connected to the silica membrane chamber through fluidic channels is not limited. Each functional chamber may individually connect to the silica membrane chamber through its own fluidic channel. However, this configuration is not preferred because the silica membrane chamber is very small and cannot provide enough space for multiple fluidic channels to dock, thereby limiting the channel size.

Furthermore, the functional chambers in the nucleic acid extraction zone are connected to the silica membrane chamber by sharing at least part of the fluid channels. The number of fluid channels directly connected to the silica membrane chamber is no more than 5, 4, 3, or 2.

Furthermore, the functional chambers in the nucleic acid extraction zone are connected to the silica membrane chamber by sharing the first fluid channel and/or the second fluid channel. The number of fluid channels directly connected to the first fluid channel and/or the second fluid channel is no fewer than 5, 4, 3, 2, or 1.

Furthermore, the sample chamber in the nucleic acid extraction region is connected to the silica membrane chamber via a single-use valve through a first fluid channel, the waste liquid chamber is connected to the silica membrane chamber through a second fluid channel, and the remaining functional chambers are connected to the first or second fluid channel through their respective fluid channels.

Furthermore, the wash buffer chamber is connected to the first fluid channel via a single-use valve; The first elution chamber is connected to the second fluid channel either directly or via a single-use valve, the second elution chamber is connected to the first fluid channel either directly or via a single-use valve. Alternatively, the first elution chamber is connected to the first fluid channel either directly or via a single-use valve, the second elution chamber is connected to the second fluid channel either directly or via a single-use valve.

Furthermore, the functional chambers in the nucleic acid detection region comprise at least one PCR reagent chamber and at least one PCR amplification chamber, and the PCR reagent chamber is connected, with or without a single-use valve, to the PCR amplification chamber and to the volumetric chamber.

Furthermore, the PCR reagent chamber and the auxiliary volumetric chamber are connected to the volumetric chamber by sharing a portion of the fluid channel.

Furthermore, the PCR reagent chambers comprise two chambers: a first PCR reagent chamber and a second PCR reagent chamber; the first PCR reagent chamber is connected to the second PCR reagent chamber via a single-use valve; and the second PCR reagent chamber is connected, with or without a single-use valve, to the PCR amplification chamber and the volumetric chamber.

Furthermore, the PCR amplification chamber comprises one or more chambers, each connected to the PCR reagent chamber through fluid channels.

Furthermore, the lysis buffer chamber is directly connected to the sample chamber and/or directly connected to the binding buffer chamber, and/or the second elution buffer chamber is directly connected to the volumetric chamber, and/or the volumetric chamber is directly connected to the auxiliary volumetric chamber and the second PCR reagent chamber, and/or the first PCR reagent chamber is directly connected to the second PCR reagent chamber, and/or the second PCR reagent chamber is directly connected to the PCR amplification chamber.

The fluid channel enables low-speed, controllable liquid flow, which is the key to achieving the silica membrane-based adsorption and elution of the present invention. A relatively low flow rate allows the liquid passing through the silica membrane to have sufficient time for adsorption and elution, thereby minimizing the gradient decline of adsorption and elution. This ensures that the silica membrane fully adsorbs nucleic acids in the liquid and that the elution buffer fully elutes the nucleic acids, thus achieving an effect similar to that of liquid flowing vertically through the silica membrane.

The "low-speed controllable flow" described in the present invention refers to the liquid flowing in the fluid channel at an average flow rate lower than 45µL/s, preferably lower than 40µL/s, 30µL/s, 20µL/s, 15µL/s, 10µL/s, or 5µL/s.

In the applicant's prior patent CN2022109249601, micro-flow channels may also be arranged between chambers of the microfluidic chip, with channel widths defined as 0.1-10 mm and lengths of 0.1-50 mm. It is recorded that liquid flows at high speed (5-20 m/s) through the micro-flow channels to improve the lysis efficiency of cells and other substances. Obviously, high-speed liquid flow in micro-flow channels cannot be applied to the present invention. Through further research, the applicant found that different structures of fluid channels have different effects on liquid flow rate. Specifically, the liquid flow rate in the channel is related to the following factors:
a. The maximum angle between the fluid-channel membrane and the plane of the microfluidic chip after the fluid channel is expanded by the liquid;
b. The width of the fluid channel;
c. The length of the fluid channel.

After applying positive pressure to the liquid storage chamber, the liquid within the chamber obtains a certain hydraulic pressure and is driven to flow. Since the elongated fluid channel is in a closed state before fluid passes through it, the liquid flowing out of the storage chamber must overcome resistance to expand the fluid channel:
First, after the fluid channel is expanded, the smaller the maximum angle between the channel membrane and the plane of the microfluidic chip, the greater the hydraulic pressure required to expand the channel under constant membrane modulus. This means greater resistance. The magnitude of the angle generally relates to the hardness of the membrane material, which is linked to the modulus of elasticity and membrane thickness. In general, a higher elastic modulus results in a smaller maximum angle, while a lower modulus results in a larger angle.

Second, the smaller the width of the fluid channel, the smaller the cross-sectional area after expansion, and the greater the hydraulic pressure and resistance required.

Third, the longer the fluid channel, the greater the overall resistance provided by the channel.

In the applicant's prior patent CN2022109249601, the micro-flow channels actually used are quite short, near the lower limit of the length range. Under higher hydraulic pressure, the liquid can flow rapidly through the channel. Clearly, this is not the flow behavior desired for the present invention.

In the present invention, appropriate selection and combination of membrane materials, fluid-channel width, and fluid-channel length can ensure that the liquid flow rate in the fluid channel meets the above-defined low-speed controllable flow, making the channel suitable for the purposes of the invention.

When common flexible membranes are used (elastic modulus 400-6000 MPa, thickness 0.05-2 mm), and considering practical product dimensions, the channel length may be set to 5-150 mm, preferably 20-100 mm. Under such dimensions, the channel width may be 0.5-2 mm, preferably 1.3-1.8 mm. It should be noted that these dimensions do not limit the structure of the fluid channel of the present invention; they merely provide feasible ranges. Any structure capable of achieving the low-speed controllable flow described herein falls within the scope of the invention.

From the above description, it can be seen that the resistance encountered by the fluid within the fluid channel during use is dynamically variable. It is challenging for the prior art to achieve slow and uniformly controlled flow of pre-filled liquid stored in a reservoir chamber of a film-based microfluidic chip through a microchannel in a repeatable manner. When using mechanical squeezing, the volume of air sealed inside the storage chamber varies with each filling, resulting in different overall chamber volumes after filling. Consequently, the same squeezing speed produces different squeezing effects. In addition, the irregular shape of the chamber causes variations in the thickness of the liquid-driving chamber during actuation, leading to significant differences in liquid flow rate inside the channel, and in some positions even causing tearing at welded joints.

In the solution of the present invention, after applying positive pressure to the liquid storage chamber, the liquid within the chamber obtains a certain hydraulic pressure and is driven to flow. Since the elongated fluid channel is in a closed state before fluid passes through it, the liquid flowing out of the storage chamber must overcome resistance to expand the fluid channel. Moreover, after the flexible membrane of the fluid channel is expanded, because the membrane in the channel region is quite narrow, the membrane deformation generates substantial resistance to fluid flow due to the increased pressure. As a result, the fluid must continuously overcome resistance as it flows, and the flow rate within the fluid channel remains slow. As liquid flows out of the storage chamber, the pressure is released, and without increasing the squeezing stroke of the squeezing mechanism, the remaining pressure may be insufficient to drive the liquid to continue flowing. At this point, the liquid stops flowing. Therefore, the squeezing device must maintain a constant pressure on the chamber to enable uniform flow, or intermittently increase the squeezing stroke to drive segmented flow of the liquid. For example, in the case of intermittent squeezing, a pulsed squeezing mode may be used. The first application of pressure to the chamber generates internal liquid pressure and initiates fluid flow. However, due to the presence of the narrow and initially closed fluid channel, the outflow rate is extremely slow, and thus the release of internal pressure is also slow. When the internal pressure decreases to a certain level (e.g., insufficient to drive fluid through the narrow fluid channel), and since the squeezing device does not increase the squeezing stroke, the chamber volume becomes smaller and the contact force between the squeezing device and the chamber wall weakens or disappears. At this moment, by increasing the squeezing stroke and applying pressure a second time, internal liquid pressure is restored, enabling continued slow outflow through the narrow fluid channel. Through such cyclic operation, the liquid can intermittently maintain a slow discharge rate through the narrow fluid channel. As the squeezing intervals become shorter, the intermittent squeezing mode effectively becomes a continuous squeezing mode.

A valve region is provided between functional chambers and fluid channels as needed. Such need includes: the functional chamber requires pre-loading of liquids. The valve region includes irreversible single-use valve structures. When such a single-use valve structure, for example, is changed from a closed state to an open state due to fluid pressure, fluid may enter the fluid channel from the functional chamber or enter the functional chamber from the fluid channel under pressure;

The present invention also provides a fluid sample processing device, comprising:
The flexible thin-film microfluidic chip described above.

Furthermore, the fluid sample processing device comprises a plurality of mechanically actuated valves, the mechanically actuated valves being configured to repeatedly and physically control the opening and closing of fluid channels that are not equipped with single-use valves, or, after a single-use valve has been opened, repeatedly and physically continue to control the opening and closing of the fluid channel corresponding to the single-use valve;
Furthermore, the device comprises one or more first squeezing mechanisms, the position and geometric configuration of each first squeezing mechanism corresponding to a functional chamber and configured to selectively apply pressure to the corresponding functional chamber to open a single-use valve or to drive the fluid into the fluid channel and toward a desired location.

Furthermore, the mechanically actuated valves comprise one or more second compressive mechanisms. Each second compressive mechanism is positioned to correspond to a valve region of the microfluidic chip and is configured to selectively apply pressure to the corresponding valve region so as to open or close the corresponding valve.

In cooperation with the pressure control mechanisms, a special control timing sequence is used to regulate the switching pattern and duration of pneumatic solenoid valves, thereby applying a banded pressure pattern slowly to the corresponding functional chambers.

The present invention also provides the use of the fluid sample processing device described above for fluid sample processing.

Furthermore, the use is for nucleic acid extraction or nucleic acid PCR amplification and detection.

The present invention also provides a nucleic acid PCR amplification detection method implemented using the fluid sample processing device described above, wherein the method comprises:
S1. Nucleic acid extraction: After passing through the nucleic acid extraction region, the nucleic acid sample yields an eluted nucleic acid solution meeting the detection standards, which is stored in the functional chamber connected to the nucleic acid quantification region;
The sample at this stage is the target nucleic acid. Cells must be dissolved or permeabilized to release the target nucleic acid and make it available for amplification and detection. If cells are lysed, the resulting lysate contains various components other than nucleic acids, including organelles, proteins (such as proteases and nucleases), carbohydrates, and lipids. These must be further purified to extract nucleic acids. Prior to loading the sample into the fluid sample processing device of the present invention, the cells may be lysed, or cell lysis may be carried out inside the sample chamber after loading (for example, by loading the sample into the chamber together with a lysis buffer). Cells may be lysed using any method known to those skilled in the art, including chemical, mechanical (e.g., ultrasonic), and/or thermal methods.

S2. Quantification of the nucleic acid eluate:
Adjust the mechanically actuated valves so that only the liquid pathway between the volumetric chamber, the chamber storing the nucleic acid eluate, and the auxiliary volumetric chamber is open. Apply a preset compressive pressure to the chamber storing the nucleic acid eluate, so that the eluate flows through the fluid channel, passes through the volumetric chamber, and enters the auxiliary volumetric chamber. At this stage, the volumetric chamber becomes filled and contains the required amount of nucleic acid eluate.

Adjust the mechanically actuated valves so that only the liquid pathway between the chambers in the nucleic acid detection region and the volumetric chamber is open. Apply a preset compressive pressure to the volumetric chamber filled with eluate, so that the nucleic acid eluate flows through the fluid channel into the chamber connected to the nucleic acid detection region.

S3. PCR amplification and detection: In the nucleic acid detection region, subject the PCR reaction solution to PCR amplification and detection to obtain the detection result.

Furthermore, the step S1 comprises the following steps:

### S1a. Reagent loading and sample addition:

Inject lysis buffer into the lysis buffer chamber of the microfluidic chip; inject nucleic acid binding buffer into the binding buffer chamber; inject protease into the sample chamber; inject wash buffer into the washing buffer chamber; inject elution buffer into at least one of the first elution buffer chamber and the second elution buffer chamber; inject the un-lysed nucleic acid sample into the sample chamber, the sample being selected from tissue homogenate, sample preservation solution, plasma, or serum.

### S1b. Sample lysis:

Adjust the mechanically actuated valves so that only the pathway between the lysis buffer chamber and the sample chamber is open. Apply a preset compressive pressure to the lysis buffer chamber to open the single-use valve and achieve liquid mixing among the lysis buffer, sample, and protease through counter-squeezing between the lysis buffer chamber and the sample chamber, thereby performing sample lysis and generating a sample reaction solution.

After sample lysis is completed, open the pathway between the binding buffer chamber and the lysis buffer chamber. Apply a preset compressive pressure to the nucleic acid binding buffer chamber to open the single-use valve and press the nucleic acid binding buffer into the lysed sample reaction solution to achieve mixing.

S1c. Nucleic acid capture: Adjust the mechanically actuated valves so that only the pathway from the sample chamber through the silica membrane chamber to the waste liquid chamber is open. Apply a preset compressive pressure to the sample chamber so that the liquid sample opens the single-use valve and flows through the fluid channel, passes through the silica membrane chamber, and enters the waste liquid chamber, completing nucleic acid capture.

S1d. Nucleic acid washing: Adjust the mechanically actuated valves so that only the pathway from the washing buffer chamber through the silica membrane chamber to the waste liquid chamber is open. Apply a preset compressive pressure to the washing buffer chamber so that the washing buffer flows through the fluid channel, passes through the silica membrane chamber, and enters the waste liquid chamber, completing nucleic acid washing.

S1e. Nucleic acid elution: Adjust the mechanically actuated valves so that only the pathway from the first and second elution buffer chambers through the silica membrane chamber is open. Apply a preset compressive pressure to the elution buffer chamber containing the elution buffer so that the buffer flows through the fluid channel, passes through the silica membrane chamber, and enters the other elution buffer chamber. Then alternately apply a preset compressive pressure to the two elution buffer chambers until the nucleic acid adsorbed on the silica membrane is completely eluted. The elution buffer carrying the nucleic acid is finally stored in the elution buffer chamber connected to the nucleic acid quantification region, completing nucleic acid extraction.

Furthermore, the step S1 further comprises the following step:
S1f. Reagent loading and sample loading: Inject PCR reaction solution into the first PCR reaction solution chamber.

The step S3 comprises the following steps:
S3a. Mixing of PCR amplification solution: After the quantified nucleic acid eluate flows into the second PCR reaction solution chamber, adjust the mechanically actuated valves so that only the pathway between the first and second PCR reaction solution chambers is open. Alternately press the first and second PCR reaction solution chambers to mix the nucleic acid eluate with the PCR reaction solution. The PCR mixture containing the nucleic acid eluate is stored in the second PCR reaction solution chamber.

S3b. PCR amplification and detection: Adjust the mechanically actuated valves so that only the pathway between the second PCR reaction solution chamber and the PCR amplification chamber is open. Apply a preset compressive pressure to the second PCR reaction solution chamber to transfer the PCR mixture into the PCR amplification chamber. Load the preset PCR thermal cycling program onto the PCR amplification chamber and perform fluorescence acquisition at predetermined temperature points to complete nucleic acid detection.

Compared with the prior art, the advantages of the present invention lie in that:
1. In the present invention, the nucleic acid extraction and capture zone comprises an elution solution having an elution function, and the nucleic acid detection zone is the detection zone used for performing the PCR reaction. During the process in which the liquid flows from the nucleic acid extraction and capture zone to the nucleic acid detection zone, it needs to be temporarily stored in the volumetric chamber and fill the volumetric chamber, thereby achieving the purpose of obtaining a quantitative amount of liquid to be mixed with PCR reaction reagents. The present invention utilizes pressure difference and the flexible film of the quantification chamber to achieve the quantification of the elution solution, and such quantified volume can be adjusted within a certain range as required.
2. The microfluidic chip of the present invention achieves nucleic acid extraction based on the silica membrane method as well as amplification detection within a sealed two-dimensional planar flexible thin-film microfluidic chip.
3. In the flexible thin-film microfluidic chip of the present invention, a fluid channel is provided. The fluid channel not only connects the functional chambers and the silica membrane chamber to form a liquid pathway, but also provides installation space for the silica membrane chamber. The silica membrane chamber is essentially arranged on one fluid channel, and each of the other functional chambers is connected to the fluid channel provided with the silica membrane chamber, or is directly connected to the silica membrane chamber through the fluid channel. The silica membrane chamber needs to exhibit as little expansion deformation as possible, and its projected area is far smaller than that of each functional chamber. In the silica membrane method, the types of liquid flowing through the silica membrane chamber in batches are relatively many; therefore, the silica membrane chamber actually becomes the core chamber. In the prior art, functional chambers are directly connected or connected through very short connecting channels. Such conventional connection methods cannot reliably connect each functional chamber with a relatively small silica membrane chamber, nor allow the liquid pathway flowing through the silica membrane chamber to be switched at will using mechanically actuated valves or other means. It is also impossible to ensure that after switching the liquid pathway, cross-contamination caused by liquid residues, rapid vibration, or movements of pipetting operations is avoided.
4. A flexible, slender, openable fluid channel is arranged between two chambers. By using the opening and closing tension of the slender channel and the continuous resistance of the long stroke, a controlled and low-speed liquid flow between the chambers is achieved. This enables, in a two-dimensional plane, sufficient adsorption and elution of nucleic acid or analytes by the silica membrane, and solves the technical problem of quantitative acquisition of nucleic acid for PCR amplification experiments.

### Description of the Drawings

FIG. 1 is a schematic structural diagram of the flexible thin-film microfluidic chip according to Embodiment 1;
FIG. 2 is a layout schematic diagram of the inlet and valve regions of the flexible thin-film microfluidic chip according to Embodiment 1;
FIG. 3 is a schematic structural diagram of region A in FIG. 2;
FIG. 4 is a partial diagram of another scheme of the flexible thin-film microfluidic chip according to Embodiment 1.

Reference numerals: laminated film (1000), liquid inlet interface (2000), first film (1001), second film (1002), fluid channel (1110), silica membrane chamber (1201), first fluid channel (1111), second fluid channel (1112), sample chamber (1301), lysis buffer chamber (1302), binding buffer chamber (1303), PCR amplification chamber (1304), pressure-relief chamber (1305), waste liquid chamber (1306), first wash buffer chamber (1307), second wash buffer chamber (1308), first elution buffer chamber (1309), second elution buffer chamber (1310), first PCR reaction reagent chamber 1311, second PCR reaction reagent chamber (1312), volumetric chamber (1313), nucleic acid quantification auxiliary chamber (1314), sample inlet (1401), lysis buffer inlet (1402), binding buffer inlet (1403), first wash buffer inlet (1407), second wash buffer inlet (1408), first elution buffer inlet (1409), first PCR reagent inlet (1411), second PCR reagent inlet (1412), positioning hole (1003).

### Detailed Description of the Embodiments

The following provides a further non-limiting detailed description of the technical solutions of the invention with reference to preferred embodiments and the accompanying drawings. In the description of the present invention, it should be understood that the terms "central," "longitudinal," "transverse," "length," "width," "thickness," "upper," "lower," "front," "rear," "left," "right," "vertical," "horizontal," "top," "bottom," "inner," "outer," "clockwise," "counterclockwise," "axial," "radial," "circumferential," and the like indicate orientations or positional relationships based on the orientations or positional relationships shown in the drawings. In addition, the terms "first" and "second" are used merely for descriptive purposes and should not be understood as indicating or implying relative importance, nor should they be understood as implicitly specifying the quantity of the indicated technical features. Thus, features designated as "first" or "second" may explicitly or implicitly include at least one such feature. In the description of the present invention, the term "plurality" means at least two, such as two, three, etc., unless otherwise explicitly defined. The embodiments described below with reference to the drawings are exemplary and are intended to illustrate the present invention rather than to limit it.

### Terminology:

Fixed bonding region: In the present invention, the fixed bonding region divides the entire laminated film into several functional chambers and fluid channels along the plane of the films.

Functional chambers: Each functional chamber can be defined as a sample chamber, lysis buffer chamber, nucleic acid capture chamber, wash buffer chamber, elution buffer chamber, PCR amplification chamber, PCR reaction reagent chamber, etc., depending on the function of the reagent contained therein.

Fluid channel: The fluid channel of the present invention may switch between two states, namely separated and adhered, and cooperates with the pressure applied to the liquid storage functional chamber to achieve a slow, uniform, or wave-like liquid flow.

Valve region: A valve region is provided between functional chambers and fluid channels as needed. Such need includes: the functional chamber requires pre-loading of liquids or solids (e.g., lyophilized powder). The valve region includes irreversible single-use valve structures. When such a single-use valve structure, for example, is changed from a closed state to an open state due to fluid pressure, fluid may enter the fluid channel from the functional chamber or enter the functional chamber from the fluid channel under pressure. The single-use valve structure may employ various weak-connection structures known in the art, which isolate the functional chamber from the fluid channel and can be destroyed under certain thermal, electromagnetic irradiation, or mechanical forces to switch from a closed to an open state. Preferably, in the valve region, the first film and the second film are separably bonded to form the single-use valve structure, which can be destroyed under fluid impact at a certain pressure, thereby changing from the closed state to the open state. For example, when a preset pressure is applied to a functional chamber containing a fluid, the fluid can break the irreversible single-use valve structure disposed between the functional chamber and the fluid channel, thereby enabling the fluid to enter the fluid channel. The single-use valve structure may physically function as a "valve" between the liquid storage chamber and the fluid channel, and these "valves" can be simply and rapidly opened through selective squeezing, making them easier to implement in a two-dimensional planar space compared with traditional valves.

Laminated film: The laminated film mainly consists of a first film and a second film laminated together. At least one of the first film and the second film is preferably a flexible film with longitudinal stretching properties. One or more fixed bonding regions and multiple functional chambers can be defined within the laminated film along the plane of the films.

In the present invention, at least one positioning hole may also be formed on the laminated film. By providing the positioning hole, an operator can more quickly and accurately position the flexible thin-film microfluidic chip onto corresponding detection equipment and more accurately perform operations such as selective squeezing of different functional chambers.

In the present invention, the first film and the second film may include any of PET film, PE film, PP film, PA film, PS film, PI film, metallized films of any of these films, composite films of any of these films with aluminum foil, combinations of multiple films, or combinations of multiple films with aluminum-foil composites. Preferably, both the first film and the second film are flexible films.

Pressure: In the present invention, the applied pressure may be provided by a force-applying mechanism (such as a squeezing mechanism) associated with functional chambers or fluid channels. In some cases, the applied pressure may also include stress generated after deformation of the flexible films among the first and/or second films.

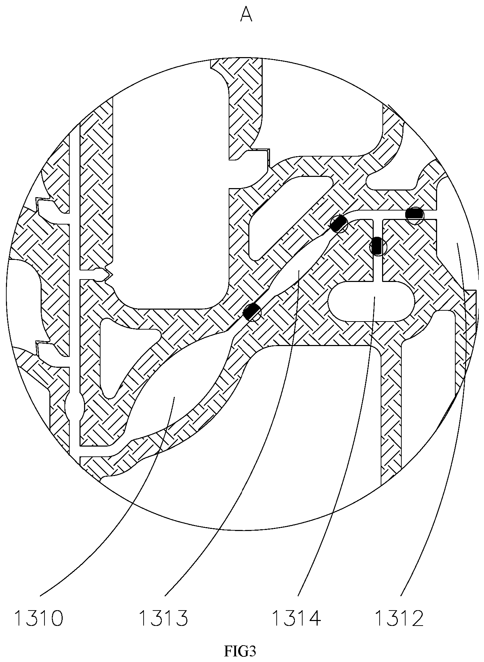

An example of controlling the opening and closing of the fluid channel and driving fluid to enter another functional chamber may be referenced from FIG. 3: First, the fluid channel between the elution buffer chamber and the volumetric chamber is opened, the fluid channel between the volumetric chamber and the nucleic acid quantification auxiliary chamber is opened, and the fluid channel between the volumetric chamber and the PCR reaction reagent chamber is blocked by a mechanically actuated valve. A first squeezing mechanism is actuated to squeeze the elution buffer chamber, allowing the liquid inside the elution buffer chamber to enter the volumetric chamber and fill the volumetric chamber, with excess liquid flowing into the nucleic acid quantification auxiliary chamber. Then, the fluid channel between the elution buffer chamber and the volumetric chamber is blocked by a mechanically actuated valve, the fluid channel between the volumetric chamber and the nucleic acid quantification auxiliary chamber is blocked by a mechanically actuated valve, and the fluid channel between the volumetric chamber and the PCR reaction reagent chamber is opened. The first squeezing mechanism is actuated to squeeze the volumetric chamber, causing all liquid within the volumetric chamber to flow into the PCR reaction reagent chamber.

### Embodiment 1

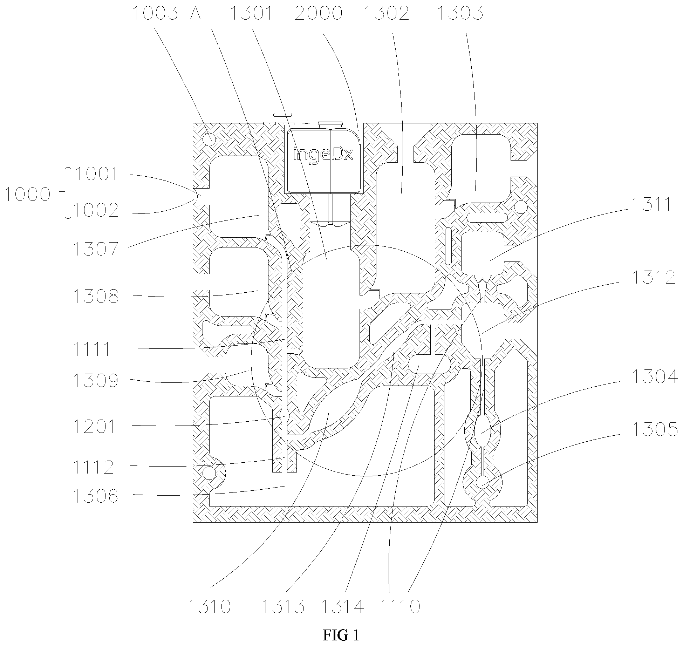

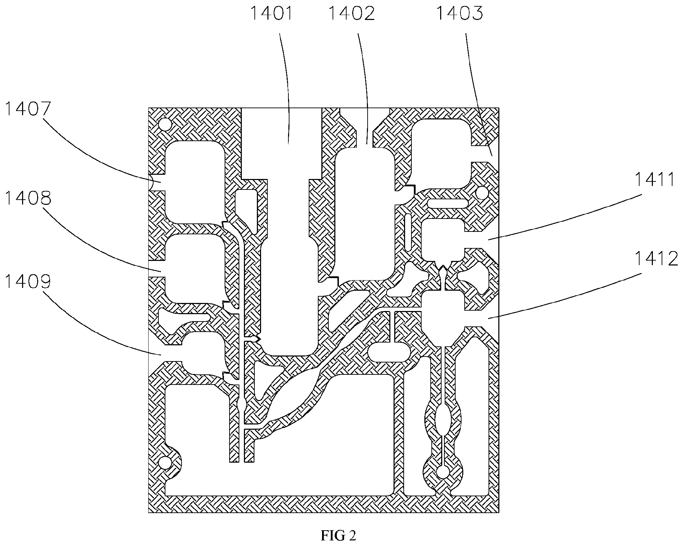

Please refer to FIG. 1-FIG. 2. This embodiment provides a flexible thin-film microfluidic chip, which mainly comprises a laminated film (1000) and a liquid-inlet interface (2000). The laminated film (1000) and the liquid-inlet interface (2000) may be joined together by thermal bonding, ultrasonic welding, chemical bonding, or other techniques to form an integrated structure.

The laminated film (1000) is mainly formed by laminating a first film (1001) and a second film (1002). At least one of the first film (1001) and the second film (1002) is preferably a flexible film with longitudinal stretchability; in this embodiment, both the first film (1001) and the second film (1002) are composite flexible films.

In this embodiment, one or more fixed bonding regions and multiple functional chambers may be defined on the laminated film (1000) along the plane of the films. Specifically, the fixed bonding regions divide the entire laminated film (1000) into several functional chambers, fluid channels (1110), and a silica membrane chamber (1201) along the plane of the films. That is, the sizes and shapes of the functional chambers, fluid channels (1110), and the silica membrane chamber (1201) are defined by the boundaries of the fixed bonding regions.

In this embodiment, the functional chambers comprise: one sample chamber (1301), one lysis buffer chamber (1302), one binding buffer chamber (1303); one PCR amplification chamber (1304), one pressure-relief chamber (1305), one waste liquid chamber (1306); two wash buffer chambers (a first wash buffer chamber (1307) and a second wash buffer chamber (1308)); two elution buffer chambers (a first elution buffer chamber (1309) and a second elution buffer chamber (1310)); two PCR reaction reagent chambers (a first PCR reaction reagent chamber (1311) and a second PCR reaction reagent chamber (1312)). The chip further comprises one volumetric chamber (1313) and one nucleic acid quantification auxiliary chamber (1314).

In this embodiment, the fluid channel (1110) may be divided into multiple sections depending on the functional chambers to which it is connected. Such sections at least comprise a first fluid channel (1111) and a second fluid channel (1112), wherein the first fluid channel (1111) is connected to the silica membrane chamber (1201), and the second fluid channel (1112) is connected to the waste liquid chamber (1314).

Among them, the sample chamber (1301), lysis buffer chamber (1302), and binding buffer chamber (1303) are connected sequentially; the sample chamber (1301) is connected to the silica membrane chamber through the first fluid channel (1111); the first wash buffer chamber (1307), second wash buffer chamber (1308), and first elution buffer chamber (1309) are each connected to the silica membrane chamber through their respective first fluid channels (1111); the waste liquid chamber (1306) is connected to the silica membrane chamber through the second fluid channel (1112). Other functional chambers are connected to the first or second fluid channel (1112) through their respective fluid channels. The volumetric chamber (1313) is connected at one end to the second elution buffer chamber (1310) and at the other end to the second PCR reaction reagent chamber (1312). The second PCR reaction reagent chamber (1312) is located between the first PCR reaction reagent chamber (1311) and the PCR amplification chamber (1304). The PCR amplification chamber (1304) is located between the second PCR reaction reagent chamber (1312) and the pressure-relief chamber (1305). The volumetric chamber (1313) is located between the second elution buffer chamber (1310) and the second PCR reaction reagent chamber (1312). The nucleic acid quantification auxiliary chamber (1314) is connected only to the volumetric chamber (1313), and communicates with the second PCR reaction reagent chamber (1312) indirectly by sharing a portion of the fluid channel with the volumetric chamber (1313).

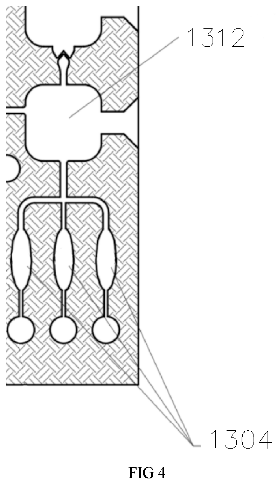

In another embodiment, referring to FIG. 4, the PCR amplification chamber (1304) is arranged as three parallel chambers, each independently connected to the second PCR reaction reagent chamber (1312) through respective fluid channels.

Each liquid storage chamber has a corresponding liquid-inlet port, such as the sample inlet port (1401), lysis buffer inlet port (1402), binding buffer inlet port (1403), first wash buffer inlet port (1407), second wash buffer inlet port (1408), first elution buffer inlet port (1409), first PCR reaction reagent inlet port (1411), and second PCR reaction reagent inlet port (1412). These inlet ports may be sealed after the corresponding liquids are filled. Among them, the sample inlet port (1401) may be connected to the liquid-inlet interface (2000).

Before injecting liquids or solids, the first film and the second film adhere closely together without any physical space in these functional chambers. After injecting the corresponding liquids or solids into the functional chambers through the sample-loading ports, the longitudinal stretchability of the flexible films allows the internal liquid pressure to cause deformation and separation of the first and second films, thus forming liquid storage chambers. When the liquid in the storage chamber is expelled due to pressure from a squeezing mechanism or the inherent stress of the flexible films, the first and second films adhere to each other again within the chamber, eliminating the physical space.

In this embodiment, one or more valve regions are defined on the laminated film (1000) along the plane of the films. Irreversible single-use valve structures are distributed within these valve regions. The valve structures are located between functional chambers and fluid channels (1110), or between functional chambers. When a single-use valve structure is changed from a closed state to an open state due to fluid pressure, fluid may enter the fluid channel from the functional chamber or enter the functional chamber from the fluid channel under pressure. In this embodiment, the first film and the second film are separably bonded within the valve regions to form the single-use valve structures. The term "separably bonded" refers to a bonding relationship that can be broken under certain conditions, such as under a certain external force, so that the first and second films separate from each other in the valve region. Preferably, within the valve region, the first film and the second film may be bonded by laser welding, ultrasonic welding, adhesive bonding, thermal bonding, etc., and such bonding may be broken under certain heat, electromagnetic irradiation, or fluid-impact forces. Generally, the width of the single-use valve structure is equal to or slightly larger than the width of the fluid channel, and much smaller than the diameter of the functional chamber. After the functional chamber and fluid channel are opened, the opening is only at the torn valve.

In this embodiment, multiple valve regions may be provided. Multiple irreversible single-use valve structures may be located at the liquid outlet positions of functional chambers requiring or potentially requiring preloaded liquids, such as the sample chamber (1301), lysis buffer chamber (1302), binding buffer chamber (1303), first wash buffer chamber (1307), second wash buffer chamber (1308), first elution buffer chamber (1309), and first PCR reaction reagent chamber (1311).

In this embodiment, three positioning holes (1003) may also be formed on the laminated film (1000).

### Embodiment 2

This embodiment provides a fluid sample processing device, which includes the flexible thin-film microfluidic chip described in Embodiment 1.

The fluid sample processing device comprises the flexible thin-film microfluidic chip of Embodiment 1, and a first squeezing mechanism placed above or below each liquid storage chamber. When liquid flow is required, the corresponding first squeezing mechanism is actuated so that the liquid in the corresponding functional chamber is subjected to pressure. Each liquid storage chamber has a corresponding valve region. When the valve (i.e., the above-mentioned irreversible single-use valve structure) inside the valve region is subjected to a fluid pressure exceeding its rupture threshold, it tears open, thereby enabling fluid communication between the liquid storage chamber and the fluid channel connected by the valve, allowing the liquid to enter the fluid channel from the liquid storage chamber.

In this embodiment, a corresponding second squeezing mechanism may be disposed above or below each valve region to function as a mechanically actuated valve. After the valve inside the valve region is damaged due to liquid extrusion, subsequent blocking can be achieved using the corresponding mechanically actuated valve. Specifically, the second squeezing mechanism may compress the corresponding valve region to form a seal, thereby cutting off fluid flow.

The fluid sample processing device further comprises a control unit, which is at least connected to the first squeezing mechanism and is configured to regulate the operating state of the first squeezing mechanism. Additionally, the second squeezing mechanism may also be connected to the control unit, and its operating state is also regulated by the control unit. The control unit may be, but is not limited to, a PLC, MCU, or computer.

Furthermore, the fluid sample processing device may include other auxiliary mechanisms, such as drive mechanisms for the squeezing mechanisms, a base, a cover plate, etc. These auxiliary mechanisms may be configured using well-known methods in the mechanical field. The drive mechanism may be, but is not limited to, a pneumatic telescopic mechanism, electromagnetic actuated telescopic mechanism, hydraulic drive telescopic mechanism, linear motor, or other mechanical drive mechanisms.

The specific mechanical structure of the fluid sample processing device can be implemented using existing technologies, such as those described in CN2022110178734.

### Embodiment 3

A method for performing nucleic acid extraction using the microfluidic chip and the fluid sample processing device described in Embodiments 1-2 may include the following steps:
(1) Sample loading and reagent injection: Inject a saliva sample into the sample chamber of the flexible thin-film microfluidic chip; inject lysis buffer into the lysis chamber; inject binding buffer into the binding chamber.

Inject wash buffer into the first and second wash chambers, respectively; inject elution buffer into the first elution chamber.

Inject reaction solution containing primers, probes, enzymes, PCR buffer, and other reagents required for PCR amplification into the first PCR reaction chamber. Seal all inlets afterward.

Preferably, 200µL of saliva sample is used; 300µL wash buffer is loaded into the first wash chamber; 100µL wash buffer into the second wash chamber; and 40µL elution buffer into the first elution chamber. The reagent volumes of the remaining chambers are not specifically limited.

(2) Lysis: Close the relevant mechanically actuated valves and open only the pathway between the sample chamber and the lysis chamber. Apply a preset squeezing pressure sequentially to the sample chamber and the lysis chamber to mix the sample with the lysis buffer, completing the lysis process. Preferably, the reaction time between sample and lysis buffer is 10 minutes.

(3) Binding: Extrude the binding chamber to push the binding buffer into the lysis chamber.

Then close the relevant mechanically actuated valves and open only the pathway between the binding chamber and the lysis chamber. Apply a preset squeezing pressure sequentially to both chambers so that the binding buffer mixes with the sample-lysis mixture, completing the binding step.

(4) Nucleic acid capture: Close the relevant mechanically actuated valves and open only the pathway from the lysis chamber to the sample chamber. Apply a preset squeezing pressure to extrude the lysis chamber so all liquid enters the sample chamber.

Then close valves and open only the pathway from the sample chamber through the silica membrane chamber to the waste liquid chamber. Apply a preset squeezing pressure to extrude the sample chamber so the sample passes through the fluid channel and through the silica membrane chamber at a flow rate of 20µL/s, after which it is discharged into the waste liquid chamber. This completes nucleic acid capture.

Preferably, the sample chamber is extruded using a Smooth Pump mode to maintain a controlled, slow flow rate through the silica membrane.

(5) Nucleic acid washing: Close the relevant mechanically actuated valves and open only the pathway from the first wash chamber through the silica membrane chamber to the waste liquid chamber. Apply a preset squeezing pressure to extrude the first wash chamber so that the wash buffer flows through the silica membrane chamber and into the waste liquid chamber via the fluid channel, completing the first nucleic acid wash.

Close the relevant mechanically actuated valves and open only the pathway from the second wash chamber through the silica membrane chamber to the waste liquid chamber. Set the extrusion pressure to extrude the second wash chamber so that the wash buffer flows through the silica membrane chamber into the waste liquid chamber via the fluid channel, completing the second nucleic acid wash.

(6) Nucleic acid elution: Close the relevant mechanically actuated valves and open only the pathway among the first elution chamber, silica membrane chamber, and second elution chamber. Apply a preset squeezing pressure to alternately extrude the first and second elution chambers multiple times.

Finally, extrude the first elution chamber so that all elution buffer is retained in the second elution chamber, completing elution. Preferably, the alternating extrusion between the first and second elution chambers is performed three times.

(7) Nucleic acid quantification: Close mechanically actuated valves and open only the pathway among the second elution chamber, the volumetric chamber, and the auxiliary quantification chamber. Apply a preset squeezing pressure to the second elution chamber until the volumetric chamber is fully filled with the liquid from the second elution chamber and excess liquid flows into the auxiliary chamber.

Close the relevant mechanically actuated valves and open only the pathway between the volumetric chamber and the second PCR reaction chamber. Extrude the quantification chamber so that all liquid flows into the second PCR reaction chamber.

(8) PCR reagent mixing: Close the relevant mechanically actuated valves and open only the pathway between the first and second PCR reaction chambers. Apply a preset squeezing pressure sequentially to both chambers multiple times. Finally, extrude the first PCR reaction chamber so that all liquid is transferred into the second PCR reaction chamber. Preferably, the alternating extrusion is performed three times.

(9) Transfer of PCR reaction reagent: Close the relevant mechanically actuated valves and open only the pathway from the second PCR reaction chamber through the PCR amplification chamber to the pressure-relief chamber. Apply a preset squeezing pressure to slowly extrude the second PCR reaction chamber so the reaction reagent gradually fills the PCR amplification chamber, displacing the original air into the pressure-relief chamber.

Then close the relevant mechanically actuated valves to seal the liquid inside the PCR amplification chamber.

(10) PCR amplification and fluorescence detection: Load the preset PCR amplification program via the computer to control the heating module for raising and lowering the temperature of the PCR amplification chamber. When fluorescence collection is required, use dedicated equipment to scan the PCR amplification chamber and complete fluorescence acquisition. The Ct value is then calculated using post-processing software, and the results are output.

In other optional embodiments, if the sample is already pre-lysed and binding buffer has already been added, the lysis and binding steps may be omitted on the basis of the present embodiment.

In other optional embodiments, the nucleic acid washing step may be reduced to a single wash on the basis of the present embodiment.

In other optional embodiments, on the basis of the present embodiment, the sample addition step is modified by adding lyophilized components such as primers, probes, and enzymes into the second PCR reaction chamber, and adding a dissolving reagent into the first PCR reaction chamber. The PCR mixing step is also modified by closing the relevant mechanically actuated valves, first storing the quantified nucleic acid-containing liquid in the second PCR reaction chamber, and then opening only the pathway between the first and second PCR reaction chambers. A compressive pressure is applied sequentially to the first and second PCR reaction chambers multiple times, thereby mixing the dissolving reagent, lyophilized components, and the quantified nucleic acid to complete the mixing process.

In other optional embodiments, on the basis of the present embodiment, primers, probes, and enzymes are stored directly in the PCR amplification chamber. After quantification, nucleic acids flow into the second PCR reaction chamber and then into the PCR amplification chamber, mixing directly with the reagents in the PCR amplification chamber to complete the mixture. This variation can be viewed as a modified combination of Steps S8 and S9.

### Embodiment 4: Experiment on Fluid-Channel Flow Rate and silica membrane Adsorption

The experimental sample was human genomic DNA at a concentration of 50 ng/µL, with a sample volume of 200 µL. The total DNA input was therefore 10,000 ng.

The nucleic acid capture step was repeated as follows: Close the mechanically actuated valves, opening only the pathway from the sample chamber through the silica membrane chamber to the waste liquid chamber. Apply a preset squeezing pressure to the sample chamber to drive 200 µL of liquid sample through the first fluid channel, across the silica membrane chamber, and through the second fluid channel into the waste liquid chamber, thereby completing nucleic acid capture. Record the time when the sample chamber is completely emptied. The captured nucleic acid is then sequentially washed and eluted, and the DNA content in the eluate is measured using Qubit or Nanodrop equipment. The measured value is then converted into DNA recovery.

In one set of experimental configurations, the total length of the fluid channel between the sample chamber and the waste liquid chamber (including the length of the silica membrane chamber) was 100 mm. Across several configurations, the channel width was varied at 1.0 mm, 1.3 mm, 1.5 mm, 1.7 mm, 1.8 mm, and 2.0 mm.

The experimental results are shown in Table 1.

**Table 1**

| Fluid-Channe 1 Width (mm) | Emptying Time for 200 µL (s) | Flow Rate (µL/s) | Estimated Velocity (mm/s) | DNA Recovery Rate |
|---|---|---|---|---|
| 1.0 | >60 | <3.33 | <4.24 | 95% |
| 1.3 | 18.53 | 10.79 | 8.13 | 93% |
| 1.5 | 9.95 | 20.10 | 11.38 | 85% |
| 1.7 | 7.38 | 27.10 | 11.95 | 70% |
| 1.8 | 5.23 | 38.24 | 15.03 | 23% |
| 2.0 | 4.19 | 47.73 | 15.20 | 16% |

The relationship used to estimate flow rate and flow velocity was: flow velocity × 3.14 × (L/2)² = flow rate, where L is the width of the fluid channel.

The above experiment demonstrates that the adsorption performance of the silica membrane varies significantly under different flow rates and flow velocities. When the flow rate exceeds approximately 45 µL/s, the adsorption efficiency of the planar silica membrane drops sharply to below 20%. In the field, a recovery rate of 20% is generally regarded as the lowest acceptable threshold for certain low-precision nucleic acid analyses. Therefore, when the flow rate exceeds 45 µL/s, it becomes essentially impossible to meet nucleic acid adsorption requirements. Through the design of the fluid channel, the present invention ensures that the flow rate within the channel is configured to maintain a flow rate sufficiently low to enable effective nucleic acid adsorption, preferably below 45 µL/s, and preferably below 40 µL/s, 30 µL/s, 20 µL/s, 15 µL/s, 10 µL/s, or 5 µL/s. This controlled low-flow condition enables the silica membrane in its planar configuration to effectively complete nucleic acid adsorption. The same principle applies to the elution process.

### Embodiment 5: Relationship Between Fluid-Channel Length and Flow Velocity

To investigate the relationship between the length of the fluid channel and the flow velocity, the following experiment was designed:
The silica membrane chamber was removed, the mechanically actuated valve was closed, and only the channel extending from the sample chamber to the waste liquid chamber was opened. A preset compressive pressure was applied to the sample chamber so that 200 µL of liquid was driven through the fluid channel and expelled into the waste liquid chamber. The time required for the sample chamber to become completely empty was recorded. The width of the fluid channel between the sample chamber and the waste liquid chamber was fixed at 1.5 mm. Across several configurations, the channel length was set to 1 mm, 2 mm, 5 mm, 10 mm, 20 mm, and 40 mm. The experimental results are shown in Table 2.

**Table 2**

| Fluid-Channel Length (mm) | Emptying Time for 200 µL (s) | Flow Rate (µL/s) | Converted Velocity (mm/s) |
|---|---|---|---|
| 1 | 1.51 | 132.45 | 74.99 |
| 2 | 2.31 | 86.58 | 49.02 |
| 5 | 3.64 | 54.95 | 31.11 |
| 10 | 4.03 | 49.63 | 28.10 |
| 20 | 4.35 | 45.98 | 26.03 |
| 40 | 4.72 | 42.37 | 23.99 |

The experimental results further show that, for lengths greater than approximately 2 mm, changes in channel length have significantly less impact on flow rate compared with changes in channel width.

The embodiments described above represent only several possible implementations of the present invention and are provided in a detailed manner. They should not be understood as limiting the scope of the invention. It should be noted that a person skilled in the art may make various modifications and improvements without departing from the spirit of the invention. All such modifications fall within the protection scope of the present invention. Therefore, the scope of protection shall be determined by the appended claims.

## Claims

1. A flexible thin-film microfluidic chip, comprising: a laminated film (1000); at least one fixed bonding region; a plurality of functional chambers; one or more fluid channels (1110); at least one valve region; and at least one fluid inlet; wherein the fixed bonding region, the functional chambers, the one or more fluid channels (1110), and the at least one valve region are distributed within the laminated film (1000); wherein sizes and shapes of the functional chambers and the one or more fluid channels (1110) are defined by boundaries of the fixed bonding region; wherein the fluid inlet is connected to at least one functional chamber and is sealed after fluid introduction; and wherein the at least one valve region is optionally disposed between the functional chambers and the one or more fluid channels (1110), and an irreversible single-use valve structure is disposed within the at least one valve region;
wherein the laminated film (1000) comprises a first film (1001) and a second film (1002) laminated together, at least one of the first film (1001) and the second film (1002) being a flexible film, and wherein, within the fixed bonding region, the first film (1001) and the second film (1002) are irreversibly bonded;
wherein, for each functional chamber, the first film (1001) and the second film (1002) are switchable, under an applied pressure, between (i) a separated state and (ii) an attached state, and wherein, in the separated state, the functional chamber accommodates a fluid;
wherein, for each of the one or more fluid channels (1110), the first film (1001) and the second film (1002) are switchable, under an applied pressure, between (i) a separated state and (ii) an attached state, and wherein, when a fluid flows out of a functional chamber under pressure, the first film (1001) and the second film (1002) of a fluid channel (1110) separate under the fluid pressure such that the fluid passes through the fluid channel (1110) and moves from one functional chamber to another;
wherein the flexible thin-film microfluidic chip comprises at least three sequentially connected functional regions comprising a nucleic acid extraction region, a nucleic acid quantification region, and a nucleic acid detection region, the nucleic acid quantification region being located between the nucleic acid extraction region and the nucleic acid detection region;
wherein each functional region comprises at least one functional chamber, and wherein the nucleic acid extraction region comprises at least one silica membrane chamber (1201);
wherein a maximum width of the silica membrane chamber (1201) is greater than a width of a fluid channel (1110), and wherein a silica membrane matching a shape of the silica membrane chamber (1201) is placed flat within the silica membrane chamber (1201); and
wherein functional chamber(s) of the nucleic acid extraction region are connected to the silica membrane chamber (1201) through fluid channel(s) (1110) that allow controlled, low-speed flow of a fluid therethrough.

2. The flexible thin-film microfluidic chip according to claim 1, wherein the nucleic acid quantification region comprises at least a volumetric chamber (1313) and an auxiliary volumetric chamber; wherein the volumetric chamber (1313) is connected, at two ends thereof, to functional chamber(s) of the nucleic acid extraction region and the nucleic acid detection region, respectively; and wherein, during a flow of a nucleic acid extract from the nucleic acid extraction region toward the nucleic acid detection region, the nucleic acid extract successively flows into the volumetric chamber (1313) and the auxiliary volumetric chamber, and after the volumetric chamber (1313) is fully filled, any nucleic acid extract exceeding a capacity thereof flows into the auxiliary volumetric chamber.

3. The flexible thin-film microfluidic chip according to claim 2, wherein the capacity of the volumetric chamber (1313) is adjustable.

4. The flexible thin-film microfluidic chip according to any one of claims 1--3, wherein the nucleic acid extraction region comprises at least one sample chamber (1301), at least one wash buffer chamber, at least one waste liquid chamber (1306), and at least two elution buffer chambers, the at least two elution buffer chambers comprising a first elution buffer chamber (1309) and a second elution buffer chamber (1310), and wherein the volumetric chamber (1313) is connected to the second elution buffer chamber (1310) either directly or via a single-use valve.

5. The flexible thin-film microfluidic chip according to claim 4, wherein the nucleic acid extraction region comprises one sample chamber (1301), two wash buffer chambers, one waste liquid chamber (1306), and two elution buffer chambers, and wherein the two wash buffer chambers comprise a first wash buffer chamber (1307) and a second wash buffer chamber (1308).

6. The flexible thin-film microfluidic chip according to claim 4 or 5, wherein fluid channel(s) (1110) in the nucleic acid extraction region are divided into sections by the silica membrane chamber (1201), and wherein the sections are classified according to functional chamber(s) to which the sections are connected, the sections comprising at least a first fluid channel (1111) and a second fluid channel (1112); wherein the first fluid channel (1111) is connected to the sample chamber (1301); and wherein the second fluid channel (1112) is connected to the waste liquid chamber (1306).

7. The flexible thin-film microfluidic chip according to any one of claims 4--6, wherein the nucleic acid extraction region further comprises at least one lysis buffer chamber (1302) and at least one binding buffer chamber (1303); wherein the lysis buffer chamber (1302) is located between the sample chamber (1301) and the binding buffer chamber (1303); wherein the lysis buffer chamber (1302) is connected to the sample chamber (1301) and the binding buffer chamber (1303), respectively, via single-use valve(s); and wherein the fluid inlet is connected to the sample chamber (1301).

8. The flexible thin-film microfluidic chip according to any one of claims 3--7, wherein the nucleic acid extraction region is connected to the silica membrane chamber (1201) by sharing at least a part of fluid channel(s) (1110), and wherein a number of fluid channel(s) (1110) directly connected to the silica.

9. The flexible thin-film microfluidic chip according to claim 6 or 7, wherein the nucleic acid extraction region is connected to the silica membrane chamber (1201) by sharing the first fluid channel (1111) and/or the second fluid channel (1112), and wherein a number of fluid channel(s) (1110) directly connected to the first fluid channel (1111) and/or the second fluid channel (1112) is no fewer than 5, 4, 3, 2, or 1.

10. The flexible thin-film microfluidic chip according to claim 6, wherein the sample chamber (1301) in the nucleic acid extraction region is connected to the silica membrane chamber (1201) via a single-use valve through the first fluid channel (1111); wherein the waste liquid chamber (1306) is connected to the silica membrane chamber (1201) through the second fluid channel (1112); and wherein remaining functional chamber(s) are connected to the first fluid channel (1111) or the second fluid channel (1112) through respective fluid channel(s) (1110).

11. The flexible thin-film microfluidic chip according to claim 6, wherein: (a) the wash buffer chamber is connected to the first fluid channel (1111) via a single-use valve; the first elution buffer chamber (1309) is connected to the second fluid channel (1112) either directly or via a single-use valve; and the second elution buffer chamber (1310) is connected to the first fluid channel (1111) either directly or via a single-use valve; or (b) the wash buffer chamber is connected to the first fluid channel (1111) via a single-use valve; the first elution buffer chamber (1309) is connected to the first fluid channel (1111) either directly or via a single-use valve; and the second elution buffer chamber (1310) is connected to the second fluid channel (1112) either directly or via a single-use valve.

12. The flexible thin-film microfluidic chip according to any one of claims 1--11, wherein the nucleic acid detection region comprises at least one PCR reagent chamber and at least one PCR amplification chamber (1304), and wherein the PCR reagent chamber is connected, with or without a single-use valve, to the PCR amplification chamber (1304) and to the volumetric chamber (1313).

13. The flexible thin-film microfluidic chip according to claim 12, wherein the PCR reagent chamber and the auxiliary volumetric chamber are connected to the volumetric chamber (1313) by sharing a portion of a fluid channel (1110).

14. The flexible thin-film microfluidic chip according to any one of claims 12--13, wherein the PCR reagent chambers comprise a first PCR reagent chamber and a second PCR reagent chamber; wherein the first PCR reagent chamber is connected to the second PCR reagent chamber via a single-use valve; and wherein the second PCR reagent chamber is connected, with or without a single-use valve, to the PCR amplification chamber (1304) and the volumetric chamber (1313).

15. The flexible thin-film microfluidic chip according to any one of claims 12--14, wherein the PCR amplification chamber (1304) comprises one or more PCR amplification chambers (1304), each connected to the PCR reagent chamber through fluid channel(s) (1110).

16. The flexible thin-film microfluidic chip according to any one of claims 7--15, wherein at least one of the following direct connections is present: (i) the lysis buffer chamber (1302) is directly connected to the sample chamber (1301); (ii) the lysis buffer chamber (1302) is directly connected to the binding buffer chamber (1303); (iii) the second elution buffer chamber (1310) is directly connected to the volumetric chamber (1313); (iv) the volumetric chamber (1313) is directly connected to the auxiliary volumetric chamber and the second PCR reagent chamber; (v) the first PCR reagent chamber is directly connected to the second PCR reagent chamber; and/or (vi) the second PCR reagent chamber is directly connected to the PCR amplification chamber (1304).

17. A fluid sample processing device, comprising the flexible thin-film microfluidic chip according to any one of claims 1--16.

18. The fluid sample processing device according to claim 17, further comprising: (i) a plurality of mechanically actuated valves configured to repeatedly and physically control opening and closing of fluid channels (1110) not equipped with single-use valves, or, after a single-use valve has been opened, to repeatedly and physically continue to control opening and closing of a fluid channel (1110) corresponding to the single-use valve; and (ii) one or more first squeezing mechanisms, each first squeezing mechanism corresponding to a functional chamber and configured to selectively apply pressure to the corresponding functional chamber to open a single-use valve or to drive a fluid into a fluid channel (1110) and toward a desired location.

19. Use of the fluid sample processing device according to claim 17 or 18 for fluid sample processing.

20. The use according to claim 19, wherein the use is for nucleic acid extraction or nucleic acid PCR amplification and detection.

21. A nucleic acid PCR amplification detection method implemented using the fluid sample processing device according to claim 17 or 18, the method comprising:
S1, nucleic acid extraction, comprising: after passing through the nucleic acid extraction region, obtaining a nucleic acid eluate meeting detection standards and storing the nucleic acid eluate in a functional chamber connected to the nucleic acid quantification region;
S2, quantification of the nucleic acid eluate, comprising: adjusting the mechanically actuated valves so that only a liquid pathway between the volumetric chamber (1313), a chamber storing the nucleic acid eluate, and the auxiliary volumetric chamber is open; applying a preset compressive pressure to the chamber storing the nucleic acid eluate such that the nucleic acid eluate flows through a fluid channel (1110), passes through the volumetric chamber (1313), and enters the auxiliary volumetric chamber, wherein the volumetric chamber (1313) becomes filled and contains a required amount of the nucleic acid eluate; then adjusting the mechanically actuated valves so that only a liquid pathway between chamber(s) in the nucleic acid detection region and the volumetric chamber (1313) is open; and applying a preset compressive pressure to the volumetric chamber (1313) filled with the nucleic acid eluate such that the nucleic acid eluate flows through the fluid channel (1110) into a chamber connected to the nucleic acid detection region; and
S3, PCR amplification and detection, comprising: in the nucleic acid detection region, subjecting the quantified nucleic acid eluate to PCR amplification and detection to obtain a detection result.

22. The method according to claim 21, wherein the step S1 comprises:
Sla, reagent loading and sample addition;
S1b, sample lysis;
S1c, nucleic acid capture;
S1d, nucleic acid washing; and
S1e, nucleic acid elution,
as set forth below:
S1a. Injecting a lysis buffer into the lysis buffer chamber (1302); injecting a nucleic acid binding buffer into the binding buffer chamber (1303); injecting a protease into the sample chamber (1301); injecting a wash buffer into the wash buffer chamber(s); injecting an elution buffer into at least one of the first elution buffer chamber (1309) and the second elution buffer chamber (1310); and injecting an un-lysed nucleic acid sample into the sample chamber (1301), the nucleic acid sample being selected from tissue homogenate, sample preservation solution, plasma, or serum;
S1b. Adjusting the mechanically actuated valves so that only a pathway between the lysis buffer chamber (1302) and the sample chamber (1301) is open; applying a preset compressive pressure to the lysis buffer chamber (1302) to open a single-use valve and to achieve liquid mixing among the lysis buffer, the nucleic acid sample, and the protease through counter-squeezing between the lysis buffer chamber (1302) and the sample chamber (1301), thereby generating a sample reaction solution; after sample lysis is completed, opening a pathway between the binding buffer chamber (1303) and the lysis buffer chamber (1302); and applying a preset compressive pressure to the nucleic acid binding buffer chamber to open the single-use valve and press the nucleic acid binding buffer into the sample reaction solution to achieve mixing;
Slc. Adjusting the mechanically actuated valves so that only a pathway from the sample chamber (1301) through the silica membrane chamber (1201) to the waste liquid chamber (1306) is open; and applying a preset compressive pressure to the sample chamber (1301) such that a liquid sample opens the single-use valve and flows through the fluid channel (1110), passes through the silica membrane chamber (1201), and enters the waste liquid chamber (1306), thereby completing nucleic acid capture;
S1d. Adjusting the mechanically actuated valves so that only a pathway from the wash buffer chamber through the silica membrane chamber (1201) to the waste liquid chamber (1306) is open; and applying a preset compressive pressure to the wash buffer chamber such that the wash buffer flows through the fluid channel (1110), passes through the silica membrane chamber (1201), and enters the waste liquid chamber (1306), thereby completing nucleic acid washing;
S1e. Adjusting the mechanically actuated valves so that only a pathway from the first elution buffer chamber (1309) and the second elution buffer chamber (1310) through the silica membrane chamber (1201) is open; applying a preset compressive pressure to an elution buffer chamber containing the elution buffer such that the elution buffer flows through the fluid channel (1110), passes through the silica membrane chamber (1201), and enters the other elution buffer chamber; and then alternately applying a preset compressive pressure to the two elution buffer chambers until nucleic acid adsorbed on the silica membrane is completely eluted, wherein the elution buffer carrying the nucleic acid is stored in the elution buffer chamber connected to the nucleic acid quantification region, thereby completing nucleic acid extraction.

23. The method according to claim 21 or 22, wherein the step S1 further comprises:
Slf, reagent loading and sample loading, comprising injecting a PCR reaction solution into the first PCR reagent chamber;
and wherein the step S3 comprises:
S3a, mixing of PCR reaction solution; and
S3b, PCR amplification and detection,
as set forth below:
S3a. After the quantified nucleic acid eluate flows into the second PCR reagent chamber, adjusting the mechanically actuated valves so that only a pathway between the first PCR reagent chamber and the second PCR reagent chamber is open; and alternately pressing the first PCR reagent chamber and the second PCR reagent chamber to mix the nucleic acid eluate with the PCR reaction solution, wherein a PCR mixture containing the nucleic acid eluate is stored in the second PCR reagent chamber;
S3b. Adjusting the mechanically actuated valves so that only a pathway between the second PCR reagent chamber and the PCR amplification chamber (1304) is open; applying a preset compressive pressure to the second PCR reagent chamber to transfer the PCR mixture into the PCR amplification chamber (1304); loading a preset PCR thermal cycling program onto the PCR amplification chamber (1304); and performing fluorescence acquisition at predetermined temperature points to complete nucleic acid detection.
